(19) Europäisches Patentamt / European Patent Office / Office européen des brevets

(11) **EP 4 593 019 A1**

(12) **EUROPEAN PATENT APPLICATION**
published in accordance with Art. 153(4) EPC

(43) Date of publication:
**30.07.2025 Bulletin 2025/31**

(21) Application number: **23881956.9**

(22) Date of filing: **27.10.2023**

(51) International Patent Classification (IPC):
**G16B 30/10** (2019.01)

(52) Cooperative Patent Classification (CPC):
**G16B 30/10**

(86) International application number:
**PCT/CN2023/127070**

(87) International publication number:
**WO 2024/088381 (02.05.2024 Gazette 2024/18)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.10.2022 CN 202211335547**

(71) Applicant: **Nanjing GenScript Biotech Co., Ltd. Nanjing, Jiangsu 211100 (CN)**

(72) Inventors:
• **HAO, Xiaohu**
  **Nanjing, Jiangsu 211100 (CN)**
• **FAN, Long**
  **Shanghai 200131 (CN)**

(74) Representative: **Wang, Bo**
**Panovision IP**
**Ebersberger Straße 3**
**85570 Markt Schwaben (DE)**

Remarks:
The complete document including Reference Table(s) and the Sequence Listing(s) can be downloaded from the EPO website

(54) **CONSTRUCTION METHOD FOR HUMANIZED ANTIBODY SEQUENCE EVALUATION MODEL AND USE THEREOF**

(57) **The** present application provides a construction method for a humanized antibody sequence evaluation model and a use thereof. **The** construction method for a humanized antibody sequence evaluation model comprises: acquiring amino acid sequences of a plurality of human-derived antibody templates and numbering the amino acid sequences; calculating an entropy value at each number position; and constructing a humanized antibody sequence evaluation model on the basis of the entropy value. A method for obtaining a candidate humanized antibody sequence by means of evaluation comprises: numbering a humanized antibody sequence to be evaluated; determining the weight and the probability of amino acid occurrence at each number position in said humanized antibody sequence; on the basis of the weight and the probability of amino acid occurrence, evaluating said humanized antibody sequence by using the humanized antibody sequence evaluation model constructed by means of the described method, to obtain an evaluation value; and if the evaluation value meets a preset condition, determining said humanized antibody sequence corresponding to the evaluation value as a candidate humanized antibody sequence. **The** present application further provides a monoclonal antibody humanization method.

EP 4 593 019 A1

Acquiring amino acid sequences of a plurality of human-derived antibody templates and numbering the amino acid sequences 201

Calculating an entropy value at each numbered position 202

Constructing a humanized antibody sequence evaluation model on the basis of the entropy value 203

FIG. 2

**Description**

**CROSS-REFERENCE TO RELATED APPLICATION**

**The** present disclosure claims the priority to Chinese Patent Application No. 202211335547.8, filed on October 28, 2022, the entire contents of which are incorporated herein by reference.

**TECHNICAL FIELD**

**The** present application relates to the technical field of biomedicine, in particular to a construction method for a humanized antibody sequence evaluation model and use thereof.

**BACKGROUND**

Monoclonal antibody drugs have advantages of strong specificity, small side effects, and remarkable curative effects in the treatment of diseases such as tumors, infectious diseases, and autoimmune diseases. At present, cell fusion and hybridoma technologies are the most reliable methods to prepare monoclonal antibodies. Animals commonly used for immunization include mice, rats, sheep/goats, rabbits, and other animals. A monoclonal antibody obtained by this method is animal-derived. To develop an animal-derived monoclonal antibody drug to be applied to the human body, humanization modification is necessary, so as to reduce the human anti-animal-derived antibody response caused by such heterologous antibodies; furthermore, the humanization also makes it possible to more effectively activate the human immune system, reduce the clearance rate of such antibody drugs, and prolong the half-life.

There are many traditional modification methods for antibody humanization, most of which are based on primary structure sequence analysis or static three-dimensional structure analysis, e.g., chimeric antibodies, CDR (complementary determining region)-grafted engineered antibodies, surface remodeled humanized antibodies, and humanized antibodies obtained by phage display. However, these are not applicable to all animal-derived antibodies. As for rabbit antibodies, direct chimerism cannot result in effective antibodies, for example, as for a variable region of a rabbit antibody that is chimerized with a constant region of a human-derived antibody, cysteine at position 80 of a light chain of the rabbit antibody forms a free disulfide bond, resulting in a polymerization reaction between the antibody light chains, leading to antibody failure. The CDR-grafted engineered antibodies and the humanized antibodies obtained by phage display are considered to be the most humanized antibodies; however, the affinity of the humanized antibodies engineered by these two methods often decreases seriously or even disappears completely. The surface remolded humanized antibodies are obtained based on the analysis of the three-dimensional structure of antibody molecules and have certain advantages in maintaining affinity over CDR-grafted antibodies; however, there are still a large number of murine-derived amino acids within the antibodies, and the maintenance of the affinity thereof is at the expense of a certain degree of humanization.

The traditional modification methods for antibody humanization can predict neither the influence of amino acid substitution on antibody structure nor the influence of amino acid change on antibody affinity during the operation of antibodies. Especially for methods based on biological experiments, the production cycle is long, the cost is high, and the internal and external environmental factors need to be strictly controlled. However, calculation-based accurate antibody structure prediction and molecular docking can quickly derive the antibody-antigen interaction after amino acid substitution and screen out effective humanized antibodies from numerous designed candidate humanized sequences, thereby resulting in a short time consumption for the antibody humanization process and a low cost.

In addition, how to quickly and conveniently screen out effective humanized antibodies from multiple candidate humanized antibody sequences is very important in antibody humanization modification. At present, it is possible to evaluate the humanized antibody sequence based on a computer simulation method; however, this method is mainly applied to murine monoclonal antibodies and cannot be applied to other species-derived monoclonal antibodies. Moreover, this method is based on machine learning or deep learning; in addition, the database used for training models is very huge, the training process is complex, and the requirements for computing resources are extremely high.

Therefore, it is necessary to establish a computational-simulation-based humanization method for monoclonal antibodies, which is applicable to all animal-derived antibodies, a method for constructing a humanized antibody sequence evaluation model, and a method for evaluating a humanized antibody based on this model. These methods can be applied to antibodies derived from all species, and have a small data size, a short time consumption, and a low cost.

**SUMMARY**

According to one aspect, the present application provides a method for constructing a humanized antibody sequence evaluation model, comprising acquiring amino acid sequences of a plurality of human-derived antibody templates and numbering the amino acid sequences; calculating an entropy value at each numbered position; and

constructing a humanized antibody sequence evaluation model on the basis of the entropy value.

In some embodiments, the entropy value is determined by a position specific scoring matrix, wherein the method further comprises: according to the numbered longest sequence, determining a multiple sequence alignment result of the amino acid sequences of the plurality of human-derived antibody templates, wherein gap positions are filled by inserting symbols; and on the basis of the multiple sequence alignment result, constructing the position specific scoring matrix of the amino acid sequences of the plurality of human-derived antibody templates.

In some embodiments, the entropy value is calculated by the following formula: $\frac{1}{n}\sum_{i=1}^{n} p_i \log p_i$, in which $n$ is the total number of the types of all amino acids and inserted symbols appearing at a certain numbered position and is maximally 21, i is an index of n, and $p_i$ is the probability of occurrence of the ith amino acid as derived from the position specific scoring matrix at this position.

In some embodiments, constructing a humanized antibody sequence evaluation model on the basis of the entropy value comprises: determining a weight at each numbered position, wherein the weight is negatively correlated with the entropy value.

In some embodiments, the weight is calculated by the following formula: $w_{pos} = \frac{e_{pos,pos=N,N-1,\ldots,1}}{\sum_{pos=1}^{N} e_{pos}}$, in which $w_{pos}$ is the weight of a numbered position, $e_{pos}$ is the entropy value at this position, and N is the numbering annotation length of the longest sequence.

In some embodiments, the humanized antibody sequence evaluation model is expressed by the following formula: $Score_{target} = \sum_{aa=1}^{N} w_{pos} p_{aa}$, in which $Score_{target}$ represents the output evaluation value, $w_{pos}$ is the weight at a certain numbered position in the humanized antibody sequence, and $p_{aa}$ represents the probability of occurrence of an amino acid at this position.

In some embodiments, the humanized antibody sequence evaluation model is used for evaluating humanized sequences of antibodies derived from all species.

According to another aspect, the present application provides a method for obtaining a candidate humanized antibody sequence by means of evaluation, comprising: numbering a humanized antibody sequence to be evaluated; determining the weight at each numbered position in the humanized antibody sequence to be evaluated and the probability of occurrence of an amino acid at this numbered position; on the basis of the weight and the probability of occurrence of the amino acid, evaluating the humanized antibody sequence to be evaluated using the humanized antibody sequence evaluation model constructed by the above method to obtain an evaluation value; and if the evaluation value meets a preset condition, determining the humanized antibody sequence to be evaluated corresponding to the evaluation value as a candidate humanized antibody sequence.

In some embodiments, the preset condition is that the evaluation value exceeds a preset threshold or is ranked higher than a certain value after ranking.

In some embodiments, the method further comprises determining a humanized antibody sequence of interest by: predicting an antibody structure of the candidate humanized antibody sequence; simulating the binding of the candidate antibody structure with a corresponding antigen to obtain the candidate antibody structure; selecting the candidate antibody structure for biological experimental validation; and determining the humanized antibody sequence of interest based on the result of the biological experimental validation.

According to still another aspect, the present application provides a method for humanizing a monoclonal antibody, comprising: determining a human-derived antibody light chain template sequence and a human-derived antibody heavy chain template sequence for the monoclonal antibody respectively; substituting CDR regions in a light chain and heavy chain of the monoclonal antibody for corresponding CDR regions in the human-derived antibody light chain template sequence and the human-derived antibody heavy chain template sequence respectively, to obtain a humanized antibody light chain template sequence and humanized antibody heavy chain template sequence resulting from the CDR region substitution; subjecting the humanized antibody light chain template sequence resulting from the CDR region substitution to an E-F ring treatment to obtain a plurality of candidate humanized antibody light chain sequences; and subjecting the humanized antibody heavy chain template sequence resulting from the CDR region substitution to a D-E ring treatment to obtain a plurality of candidate humanized antibody heavy chain sequences.

In some embodiments, subjecting the humanized antibody light chain template sequence resulting from the CDR region substitution to an E-F ring treatment to obtain a plurality of candidate humanized antibody light chain sequences comprises: performing a search in a database for the humanized antibody light chain template sequences resulting from the CDR region substitution to obtain a multiple sequence alignment result; according to the multiple sequence alignment result, constructing a position specific scoring matrix for E-F rings; generating a plurality of E-F ring sequences according to the position specific scoring matrix for E-F rings; and substituting the plurality of E-F ring sequences for the corresponding sequence in the humanized antibody light chain template sequence resulting from the CDR region substitution to obtain the plurality of candidate humanized antibody light chain sequences.

In some embodiments, subjecting the humanized antibody heavy chain template sequence resulting from the CDR region substitution to a D-E ring treatment to obtain a plurality of candidate humanized antibody heavy chain sequences comprises: performing a search in a database for the humanized antibody heavy chain template sequences resulting from the CDR region substitution to obtain a multiple sequence alignment result; according to the multiple sequence alignment result, constructing a position specific scoring matrix for D-E rings; generating a plurality of D-E ring sequences according to the position specific scoring matrix for D-E rings; and substituting the plurality of D-E ring sequences for the corresponding sequence in the humanized antibody heavy chain template sequence resulting from the CDR region substitution to obtain the plurality of candidate humanized antibody heavy chain sequences.

In some embodiments, the method further comprises: subjecting highly conserved sites in the plurality of candidate humanized antibody light chain sequences and the plurality of candidate humanized antibody heavy chain sequences respectively to back mutation.

In some embodiments, subjecting highly conserved sites in the plurality of candidate humanized antibody light chain sequences and the plurality of candidate humanized antibody heavy chain sequences respectively to back mutation comprises: determining highly conserved sites in the light chain and heavy chain sequences of the monoclonal antibody respectively; determining whether the plurality of candidate humanized antibody light chain sequences and the plurality of candidate humanized antibody heavy chain sequences are respectively consistent with the amino acids of the light chain and heavy chain of the monoclonal antibody at the highly conserved sites; and if not consistent, substituting the amino acids at the highly conserved sites in the plurality of candidate humanized antibody light chain sequences and the plurality of candidate humanized antibody heavy chain sequences back with the amino acids at the corresponding positions in the light chain and heavy chain of the monoclonal antibody.

In some embodiments, the monoclonal antibody is a rabbit monoclonal antibody, and the method further comprises: predicting three-dimensional structures of a light chain and a heavy chain of the rabbit monoclonal antibody; according to the predicted three-dimensional structures, if there are a pair of cysteines in the rabbit monoclonal antibody, with the distance between this pair of cysteines being between 4 and 7 angstroms, and no amino acid is present at the corresponding positions in the humanized antibody light chain template sequence and the humanized antibody heavy chain template sequence resulting from the CDR region substitution, inserting serine at the positions, with the positions excluding the CDR region; and if there is only a single cysteine in the rabbit monoclonal antibody and no amino acid is present at the corresponding position in the humanized antibody light chain template sequence and the humanized antibody heavy chain template sequence resulting from the CDR region substitution, inserting serine at the position.

In some embodiments, the method further comprises: evaluating the light chain sequences and heavy chain sequences of the candidate humanized antibodies respectively by the humanized antibody sequence evaluation model constructed using the above method to obtain light chain sequences and heavy chain sequences of humanized antibodies.

In some embodiments, the method further comprises: subjecting the humanized antibodies to biological experimental validation to determine the humanized antibody sequence of interest.

## BRIEF DESCRIPTION OF THE DRAWINGS

FIG. 1 is a flowchart of a method for humanizing a monoclonal antibody as shown in some embodiments according to the present application;

FIG. 2 is a flowchart of a method for constructing a humanized antibody sequence evaluation model as shown in some embodiments according to the present application;

FIG. 3 is a flowchart of a method for obtaining a candidate humanized antibody sequence by means of evaluation as shown in some embodiments according to the present application;

FIG. 4A shows the classification results of distinguishing human- and murine-derived antibody sequences using the humanized antibody sequence evaluation model and an ROC curve (heavy chain);

FIG. 4B shows the classification results of distinguishing human- and murine-derived antibody sequences using the humanized antibody sequence evaluation model and an ROC curve (light chain);

FIG. 5A shows the classification results of distinguishing human- and other species-derived antibody sequences using the humanized antibody sequence evaluation model and an ROC curve (heavy chain);

FIG. 5B shows the classification results of distinguishing human- and other species-derived antibody sequences using the humanized antibody sequence evaluation model and an ROC curve (light chain); and

FIG. 6 is the results of an ELISA binding experiment of humanized antibodies from clone 81E11.

**DETAILED DESCRIPTION**

For a clearer description of the technical solutions in the embodiments of the present application, a brief introduction will be given below for the accompanying drawings required for describing the embodiments. Apparently, the accompanying drawings in the following description show merely some embodiments of the present application, and those of ordinary skill in the art may still apply the present application to other similar scenarios according to these accompanying drawings without any creative effort. Unless obvious from the linguistic context or otherwise stated, the same reference signs in the drawings represent the same structure or operation.

**As** shown in the present application and the claims, the words "one", "a", "an" and/or "the" do not specifically refer to the singular, but may also include the plural, unless the context clearly indicates otherwise. Generally, the terms "including" and "comprising" only imply the inclusion of explicitly identified steps and elements, and these steps or elements do not constitute an exclusive list. A method may further comprise other steps or elements.

Flowcharts are used in the present application to illustrate operations performed by a system according to the embodiments of the present application. It should be understood that preceding or following operations are not necessarily performed in an exact order. Instead, all steps may be executed in a reverse order or simultaneously. Moreover, it is possible to add other operations to these processes, or remove a step or several steps from these processes.

**The** present application provides a method for humanizing a monoclonal antibody. The light and heavy chains of monoclonal antibodies from any animal source were analyzed, designed, and processed based on important sites and positions of sequences to obtain humanized sequences.

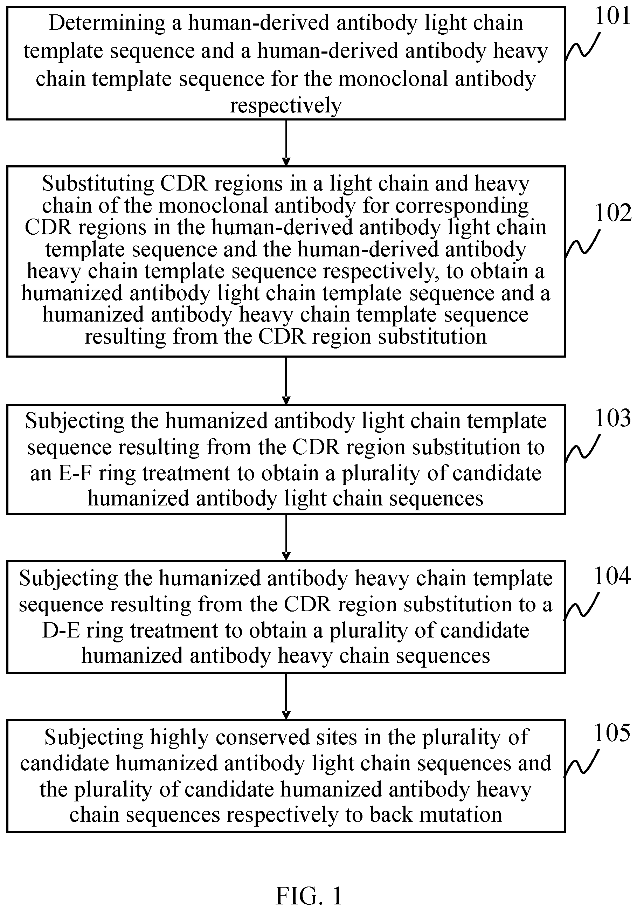

FIG. 1 is a flowchart of a method for humanizing a monoclonal antibody as shown in some embodiments according to the present application. As shown in FIG. 1, the method comprises the following steps. The method is carried out by a first computing device. In some embodiments, the computing device may comprise a processing device (or processor), a memory, an input/output interface, and a communication port. The processing device can execute computational instructions (program codes) and perform the method steps described in the present application. Computational instructions may include programs, objects, components, data structures, procedures, modules, and functions (functions refer to specific functions described in the present application). In some embodiments, the processing device may include microcontrollers, microprocessors, reduced instruction set computers (RISCs), application-specific integrated circuits (ASICs), application-specific instruction set processors (ASIPs), central processing units (CPUs), graphics processing units (GPUs), physical processing units (PPUs), microcontroller units, digital signal processors (DSPs), field programmable gate arrays (FPGAs), advanced RISC machines (ARMs), programmable logic devices, any circuits and processors capable of performing one or more functions, etc., or any combination thereof.

Step 101. A human-derived antibody light chain template sequence and a human-derived antibody heavy chain template sequence of the monoclonal antibody are determined respectively.

In some embodiments, the monoclonal antibody can be a monoclonal antibody derived from any animal. In some embodiments, the monoclonal antibody can be a murine-derived monoclonal antibody, a rabbit-derived monoclonal antibody, etc. According to the sequence of the monoclonal antibody, a search can be performed in a database (e.g., Observed Antibody Space Database (OAS)) that has collected human-derived antibody sequences and the sequence similarity can be calculated to obtain human-derived antibody light chain and heavy chain template sequences. For example, the amino acid sequences of the variable regions of the light chain and heavy chain of the monoclonal antibody can be used as an input, and a search is performed in the OAS database by using BLAST tool to obtain the amino acid sequences of the human-derived antibody light chain and heavy chain templates, respectively. In some embodiments, the human-derived antibody light chain template sequence or heavy chain template sequence may include one or more. For example, the human-derived antibody light chain or heavy chain template sequence can be a sequence with the highest similarity to the amino acid sequence of the light chain or heavy chain of the monoclonal antibody. For another example, the human-derived antibody light chain or heavy chain template sequence can be a sequence with a similarity to the amino acid sequence of the light chain or heavy chain of the monoclonal antibody that meets a certain condition (e.g., more than 98%, 95%, or 90%).

Step 102. CDR regions in a light chain and heavy chain of the monoclonal antibody are substituted for corresponding CDR regions in the human-derived antibody light chain template sequence and the human-derived antibody heavy chain template sequence respectively, to obtain a humanized antibody light chain template sequence and a humanized antibody heavy chain template sequence resulting from the CDR region substitution.

After the humanized antibody light chain template sequence and the humanized antibody heavy chain template sequence are obtained, the light chain sequence and the heavy chain sequence of the monoclonal antibody, and the humanized antibody light chain template sequence and the humanized antibody heavy chain template sequence can be numbered using a numbering system, so as to determine the CDRs, framework regions, residues affecting antibody-antigen binding affinity and antibody specificity, etc., in each sequence, so that sequence or site substitution treatment in the CDR region and non-CDR region can be performed on the monoclonal antibody sequence and the humanized antibody template sequences. In some embodiments, these sequences can be numbered uniformly using ANARCI unified numbering system. In some embodiments, Chothia numbering scheme can be used for numbering. In some embodiments, Kabat, Martin, Gelfand, IMGT, or Honneger's numbering scheme may also be used. In some embodiments, the variable region of each antibody sequence can be numbered. These sequences are aligned to the same coordinate system, and gap positions can be filled by inserting symbols, e.g., "-" and "*". Based on the numbering, the CDR regions of the light chain sequence and the heavy chain sequence of the monoclonal antibody, and the CDR regions of the humanized antibody light chain template sequence and the humanized antibody heavy chain template sequence can be marked, accordingly the CDR regions in the light chain sequence and the heavy chain sequence of the monoclonal antibody are respectively substituted for the CDR regions in the human-derived antibody light chain template sequence and the human-derived antibody heavy chain template sequence to obtain a humanized antibody light chain template sequence and a humanized antibody heavy chain template sequence resulting from the CDR region substitution.

Step 103. The humanized antibody light chain template sequence resulting from the CDR region substitution is subjected to an E-F ring treatment to obtain a plurality of candidate humanized antibody light chain sequences.

An E-F ring is a ring structure located in the non-binding region of the light chain of the antibody, which can function to support the whole antibody. Subjecting the humanized antibody light chain template sequence resulting from the CDR region substitution to an E-F ring treatment refers to substituting the E-F ring in the humanized antibody light chain template sequence resulting from the CDR region substitution with an additionally generated E-F ring sequence. Specifically, this is implemented by the following method.

In some embodiments, a search is performed in a database (e.g., UniRef90 database or UniRef 100 database) for the humanized antibody light chain template sequences resulting from the CDR region substitution to obtain a Multiple Sequence Alignment (MSA) result. It should be noted that a search may also be performed in a database for the human-derived antibody light chain template sequences to obtain a multiple sequence alignment result. According to the multiple sequence alignment result, a Position Specific Scoring Matrix (PSSM) for E-F rings is constructed. The PSSM for E-F rings indicates the possibility of occurrence of each amino acid at each position of an E-F ring. As an example, in the PSSM, the number of rows is 20, which corresponds to 20 different amino acids, and the number of columns is the length of the E-F ring sequence. According to the position specific scoring matrix for E-F rings and based on the probability of occurrence of an amino acid corresponding to each position, a plurality of E-F ring sequences can be generated. In some embodiments, a plurality of E-F ring sequences can be randomly generated according to the position specific scoring matrix. By substituting the plurality of E-F ring sequences for the corresponding sequence in the humanized antibody light chain template sequence resulting from the CDR region substitution, the plurality of candidate humanized antibody light chain sequences can be obtained. It should be noted that the probability of occurrence of an amino acid in each position may also be determined by other methods, for example, hidden Markov model.

Step 104. The humanized antibody heavy chain template sequence resulting from the CDR region substitution is subjected to a D-E ring treatment to obtain a plurality of candidate humanized antibody heavy chain sequences.

A D-E ring is a ring structure located in the non-binding region of the heavy chain of the antibody, which can function to support the whole antibody. Subjecting the humanized antibody heavy chain template sequence resulting from the CDR region substitution to a D-E ring treatment refers to substituting the D-E ring in the humanized antibody heavy chain template sequence resulting from the CDR region substitution with an additionally generated D-E ring sequence. The implementation method therefor is similar to that in step 103. Specifically, a search is performed in a database (e.g., UniRef90 database) for the humanized antibody heavy chain template sequences resulting from the CDR region substitution to obtain a multiple sequence alignment result. It should be noted that a search may also be performed in a database for the human-derived antibody heavy chain template sequences to obtain a multiple sequence alignment result. According to the multiple sequence alignment result, a position specific scoring matrix for D-E rings is constructed; a plurality of D-E ring sequences are generated according to the position specific scoring matrix for D-E rings; and the plurality of D-E ring sequences are substituted for the corresponding sequence in the humanized antibody heavy chain template sequence resulting from the CDR region substitution to obtain the plurality of candidate humanized antibody heavy chain sequences.

By obtaining a plurality of E-F ring and D-E ring sequences and substituting these sequences for the E-F ring and D-E ring in the humanized antibody light chain and heavy chain template sequences resulting from the CDR region substitution in steps 103 and 104, candidate humanized antibody light chain and heavy chain sequences with a higher degree of humanization can be obtained, which can better maintain the antibody structure and provide more candidates for obtaining the humanized antibody light chain and heavy chain sequences of interest.

Step 105. Highly conserved sites in the plurality of candidate humanized antibody light chain sequences and the plurality of candidate humanized antibody heavy chain sequences are respectively subjected to back mutation.

A highly conserved site refers to a site that is relatively important for maintaining the structure of a monoclonal antibody. If the amino acids in such sites in the light and heavy chain sequences of a candidate humanized antibody are inconsistent with those in the monoclonal antibody, the antibody structure of such a candidate humanized antibody may be changed, resulting in the decrease or even disappearance of the affinity of the antibody. Therefore, it is necessary to make them consistent with those in the corresponding sites of the monoclonal antibody. Specifically, highly conserved sites in the light chain and heavy chain sequences of the monoclonal antibody are determined first; whether the plurality of candidate humanized antibody light chain sequences and the plurality of candidate humanized antibody heavy chain sequences are respectively consistent with the amino acids of the light chain and heavy chain of the monoclonal antibody at the highly conserved sites is determined; and if not consistent, the amino acids at the highly conserved sites in the plurality of candidate humanized antibody light chain sequences and the plurality of candidate humanized antibody heavy chain sequences are substituted back with the amino acids at the corresponding positions in the light chain and heavy chain of the monoclonal antibody.

In some embodiments, the highly conserved sites are determined in a manner similar to that for the above E-F ring sequence and can be determined by a PSSM of the light and heavy chains of the monoclonal antibody. The PSSM can be obtained by inputting the light and heavy chains of the monoclonal antibody into a protein database and performing a search.

Additionally or alternatively, where the monoclonal antibody is a rabbit monoclonal antibody, the method may further comprise subjecting the rabbit monoclonal antibody to a cysteine treatment. Since the variable region of the rabbit monoclonal antibody is chimerized with a constant region of a human-derived antibody, cysteine at position 80 of a light chain of the rabbit antibody can form a free disulfide bond, resulting in a polymerization reaction between the antibody light chains, leading to antibody failure. Therefore, it is necessary to treat cysteine at the corresponding position in the template sequence to avoid antibody failure. Specifically, the three-dimensional structures of the light chain and heavy chain of the rabbit monoclonal antibody are predicted; according to the predicted three-dimensional structures, if there are a pair of cysteines in the rabbit monoclonal antibody, with the distance between this pair of cysteines being between 4 and 7 angstroms, and no amino acid is present at the corresponding positions in the humanized antibody light chain template sequence and humanized antibody heavy chain template sequence resulting from the CDR region substitution, serine is inserted at the positions, with the positions excluding the CDR region; if there is only a single cysteine in the rabbit monoclonal antibody and no amino acid is present at the corresponding position in the humanized antibody light chain template sequence and the humanized antibody heavy chain template sequence resulting from the CDR region substitution, serine is inserted at the positions.

It should be noted that the above description of the method steps with reference to FIG. 1 and the sequence of the steps are only for illustration and explanation and do not limit the scope of application of the present application. For those skilled in the art, various modifications and changes can be made to the steps under the guidance of the present application. However, these modifications and changes are still within the scope of the present application. For example, steps 103 and 104 can be combined into one step or have their order swapped. For example, step 103 may follow step 104.

**The** present application further provides a method for constructing a humanized antibody sequence evaluation model. By means of the above method, an animal-derived monoclonal antibody can be humanized and a plurality of candidate humanized antibodies can be obtained. Subsequently, a humanized antibody sequence evaluation model can be constructed based on the method described below. By means of this model, the plurality of candidate humanized antibodies can be evaluated and screened, and an effective humanized antibody of interest can be screened out.

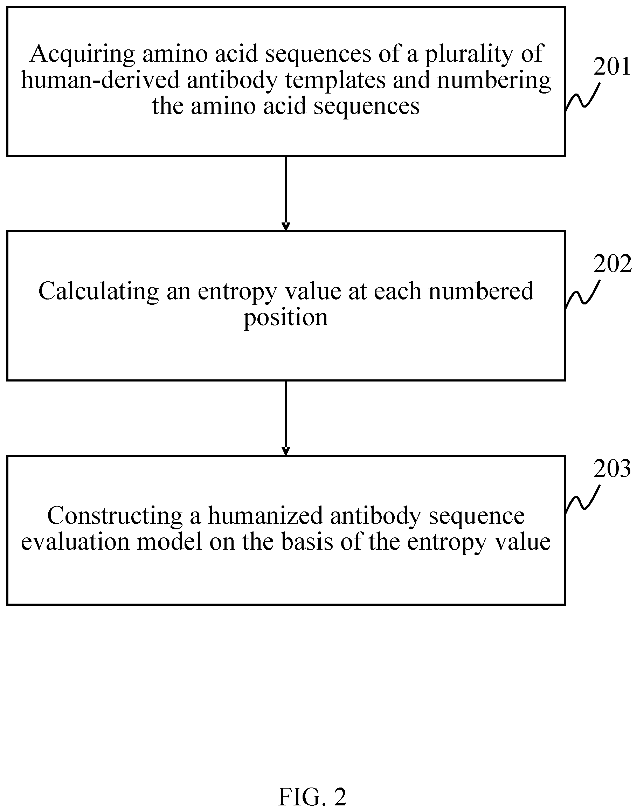

FIG. 2 is a flowchart of a method for constructing a humanized antibody sequence evaluation model as shown in some embodiments according to the present application. As shown in FIG. 2, the method comprises the following steps. The method is carried out by a second device.

Step 201. Amino acid sequences of a plurality of human-derived antibody templates are acquired and the amino acid sequences are numbered.

In some embodiments, the amino acid sequences of the human-derived antibody templates can be acquired from a human-derived antibody database. The database may be an OAS database. In some embodiments, the amino acid sequences of the human-derived antibody templates may be all or some of the sequences of the database. The amino acid sequences of the human-derived antibody templates can be numbered by using a numbering system. In some embodiments, the numbering method may be the same as that in step 102. In some embodiments, the amino acid sequences of the human-derived antibody templates can be variable region sequences or whole antibody sequences obtained from the database. In the model construction method, the explanation is based on variable region sequences.

Step 202. An entropy value at each numbered position is calculated.

In some embodiments, the entropy value is determined by a position specific scoring matrix. The method may further comprise constructing the position specific scoring matrix of the amino acid sequences of the plurality of human-derived antibody templates. In some embodiments, constructing the position specific scoring matrix of the amino acid sequences of the plurality of human-derived antibody templates comprises: according to the numbered longest sequence, determining a multiple sequence alignment result of the amino acid sequences of the plurality of human-derived antibody templates, wherein gap positions are filled by inserting symbols; and on the basis of the multiple sequence alignment result, constructing the position specific scoring matrix of the amino acid sequences of the plurality of human-derived antibody templates. The construction of the position specific scoring matrix of the amino acid sequences of the human-derived antibody templates is similar to the construction of the position specific scoring matrix of the E-F ring in step 103 in FIG. 1 and is no more repeated here.

**The** position specific scoring matrix indicates the score of the possibility of occurrence of each amino acid at each numbered position. Based on the position specific scoring matrix, the probability of occurrence of each amino acid can be determined, and thus, the entropy value at each numbered position can be determined. The entropy value is used to indicate the stability of the amino acid at each numbered position. The higher the entropy value, the more unstable the amino acid at this position, the greater the change of the amino acid, and the less the conservativeness.

In some embodiments, the entropy value is calculated by the following formula (1):

$$\frac{1}{n}\sum_{i=1}^{n} p_i \, log p_i, \ (1)$$

in which n is the total number of the types of all amino acids and inserted symbols appearing at a certain numbered position and is maximally 21, i is an index of n, and $p_i$ is the probability of occurrence of the ith amino acid at this position and can be derived from the position specific scoring matrix.

In some embodiments, the entropy value is determined by hidden Markov model. For example, the probability of occurrence of an amino acid at each position can be determined by hidden Markov model, and the entropy value can be determined based on the probability of occurrence.

Step 203. A humanized antibody sequence evaluation model is constructed on the basis of the entropy value.

In some embodiments, constructing a humanized antibody sequence evaluation model on the basis of the entropy value comprises: determining the weight at each numbered position. The weight is negatively correlated with the entropy value. The greater the entropy value, the more unstable the amino acid at this position, and the lower the importance. Therefore, if the weight is assigned in a reverse way, the corresponding weight will be lower.

In some embodiments, the weight is calculated by the following formula (2):

$$w_{pos} = \frac{e_{pos,pos=N,N-1,\dots,1}}{\sum_{pos=1}^{N} e_{pos}}, \ (2)$$

in which $w_{pos}$ is the weight of a numbered position, $e_{pos}$ is the entropy value at this position and determined by formula (1), and N is the numbering annotation length of the longest sequence.

In some embodiments, the humanized antibody sequence evaluation model is expressed by the following formula (3):

$$\text{Score}_{target} = \sum_{aa=1}^{N} w_{pos} p_{aa}, \ (3)$$

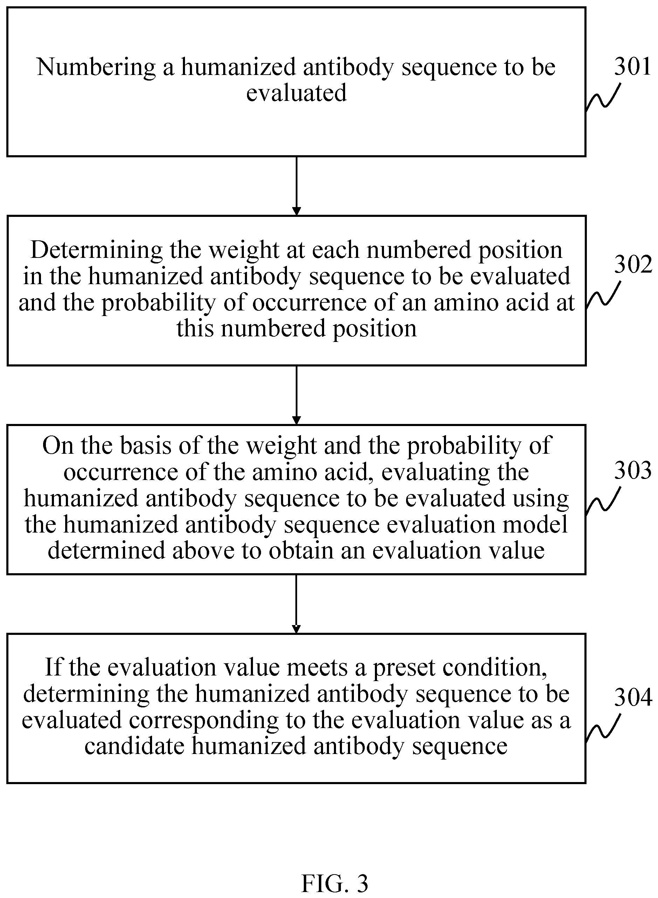

in which $Score_{target}$ represents the output evaluation value, $w_{pos}$ is the weight at a certain numbered position in the humanized antibody sequence, and $p_{aa}$ represents the probability of occurrence of an amino acid at this position and is determined according to the position specific scoring matrix. In some embodiments, the humanized antibody sequence evaluation model is used for evaluating humanized sequences of antibodies derived from all species. For the specific evaluation method, reference can be made to FIG. 3 and the description part thereof below.

In some embodiments, through the validation of the model, the humanized antibody sequence evaluation model can effectively distinguish the light chain of a human-derived antibody sequence from that of a murine-derived antibody sequence, with the corresponding AUC value being 1.00; and the humanized antibody sequence evaluation model can distinguish the heavy chain of the human-derived antibody sequence from that of the murine-derived antibody sequence, with the corresponding AUC value being 0.92. The humanized antibody sequence evaluation model can distinguish the light chain of a human-derived antibody sequence from that of other species-derived antibody sequences, with the corresponding AUC value being 0.89; and the humanized antibody sequence evaluation model can distinguish the heavy chain of a human-derived antibody sequence from that of other species-derived antibody sequences, with the corresponding AUC value being 0.94. These results indicate that the humanized antibody sequence evaluation model of the present application can effectively distinguish humanized antibody sequences from other species-derived antibody sequences and can be applied to the subsequent evaluation and screening of candidate antibodies.

**The** present application further provides a method for obtaining a candidate humanized antibody sequence by means of evaluation. In this method, based on the humanized antibody sequence evaluation model constructed above, the humanized antibody sequences to be evaluated are evaluated to determine a humanized antibody sequence of interest.

FIG. 3 is a flowchart of a method for obtaining a candidate humanized antibody sequence by means of evaluation as shown in some embodiments according to the present application. As shown in FIG. 3, the method mainly comprises the following steps. The method is mainly carried out by a third device.

Step 301. A humanized antibody sequence to be evaluated is numbered.

In some embodiments, the humanized antibody sequence to be evaluated can be numbered according to the above-mentioned numbering method. In some embodiments, the humanized antibody sequence to be evaluated can be determined based on the method shown in FIG. 1.

Step 302. The weight and the probability of occurrence of an amino acid at each numbered position in the humanized antibody sequence to be evaluated are determined.

In some embodiments, in order to determine the weight at each numbered position in the humanized antibody sequence to be evaluated, it is necessary to determine the entropy value at each numbered position in the humanized antibody sequence to be evaluated. The entropy value is determined according to the position specific scoring matrix of a plurality of human-derived antibody template sequences. The entropy value and weight are determined by the above formulas (1) and (2), respectively.

Step 303. On the basis of the weight and the probability of occurrence of the amino acid, the humanized antibody sequence to be evaluated is evaluated using the humanized antibody sequence evaluation model constructed by the above method to obtain an evaluation value. Specifically, according to the position specific scoring matrix of the plurality of human-derived antibody template sequences, the probability of occurrence of an amino acid at each numbered position in each humanized antibody sequence to be evaluated is determined. The weight and the probability of occurrence of the amino acid are input into the humanized antibody sequence evaluation model, namely formula (3), and the model can output the evaluation value of each humanized antibody sequence to be evaluated.

Step 304. If the evaluation value meets a preset condition, the humanized antibody sequence to be evaluated corresponding to the evaluation value is determined as a candidate humanized antibody sequence. In some embodiments, the preset condition may be that the evaluation value is greater than a preset threshold (e.g., 0.5, 0.6, or 0.7). For example, the humanized antibody sequence to be evaluated with the evaluation value being greater than 0.6 is determined as a candidate humanized antibody sequence. In some embodiments, the evaluation values can be ranked, for example, in a positive sequence, and the preset condition can be that the ranking is higher than a certain value (e.g., 3, 4, 5, 6, 7, 8, 9, 10, 11, 12, 13, 14, or 15). For example, the humanized antibody sequences to be evaluated corresponding to the top 15 evaluation values are determined as candidate humanized antibody sequences.

Additionally or alternatively, the evaluation method may further comprise determining a humanized antibody sequence of interest by: predicting an antibody structure of the candidate humanized antibody sequence; simulating the binding of the candidate antibody structure with a corresponding antigen to obtain the candidate antibody structure; selecting the candidate antibody structure for biological experimental validation; and determining the humanized antibody sequence of interest based on the result of the biological experimental validation. Specifically, the light and heavy chains of the candidate humanized antibody sequences are paired in pairs, and the candidate antibody structures after pairing are predicted using an antibody structure prediction software. An antigen structure is predicted by an antigen structure prediction software. The candidate antibody structures and the antigen structure are simulated by means of computational simulation, and the candidate antibody structures with better simulation effects are selected for the subsequent biological experimental validation to detect the affinity, so as to determine a humanized antibody sequence of interest.

Only by way of example, a gene of the candidate humanized antibody sequence is synthesized and constructed into eukaryotic expression vectors containing light chain and heavy chain conserved regions, so that the vectors can express a complete antibody molecule. Subsequently, the light chain and heavy chain vectors are co-transfected into 293 or CHO cells for transient expression, and the supernatant from the transfected cell culture is collected. The binding of the candidate antibody to the antigen is validated by ELISA, and the antibodies positive for binding are further subjected to antibody purification by using Protein A. Subsequently, the antibodies are subjected to affinity detection by a technique such as ELISA, FACS or SPR and compared with the parent antibody to get the affinity result.

It should be noted that the above description of the method steps with reference to FIGS. 1, 2, and 3 and the sequence of the steps are only for illustration and explanation and do not limit the scope of application of the present application. For those skilled in the art, various modifications and changes can be made to the steps under the guidance of the present application. However, these modifications and changes are still within the scope of the present application. In some embodiments, the first computing device, the second computing device, and the third computing device may be independent computing devices or may be merged into one computing device.

**The** humanized monoclonal antibodies can be effectively obtained by the method for humanizing a monoclonal antibody according to the present application; and according to the constructed humanized antibody sequence evaluation model based on the entropy value, the humanized monoclonal antibodies can be evaluated, so as to screen out effective humanized antibodies.

**EXAMPLES**

**Example 1 Construction of a humanized antibody sequence evaluation model**

A model was constructed by using human-derived antibody sequences in OAS database, tested, and evaluated. The OAS database stored more than 2.5 billion antibody sequence data including those of various species such as human, mouse, and monkey. 5,000,000 human-derived antibody sequences were randomly extracted from the OAS database, and a humanized antibody sequence evaluation model was constructed as follows.

**All** the 5,000,000 human-derived antibody sequences obtained from the OSA database were numbered and annotated by ANARCI unified numbering system.

According to the numbering annotation of the longest sequence in the numbering annotation, the Multiple Sequence Alignment (MSA) result of all the human-derived antibody sequences was constructed, wherein gap positions in shorter sequences were filled by inserting "-".

According to the obtained MSA, the Position Specific Scoring Matrix (PSSM) of all the human-derived antibodies was constructed.

According to the obtained PSSM, the entropy value was calculated for each position in the numbering annotation.

The entropy value calculation formula is as follows: $\frac{1}{n}\sum_{i=1}^{n} p_i \log p_i$ , in which n is the total number of the types of all amino acids and the inserted "-" appearing at a certain numbered position and is maximally 21, i is an index of n, and $p_i$ is the probability of occurrence of the ith amino acid as derived from PSSM at this position.

The calculated entropy value at each position was normalized, and according to the normalized entropy value, the weight was assigned to each position in a reverse way, that is, the position with the highest entropy value was assigned with the minimum weight, and the position with the lowest entropy value was assigned with the highest weight. The weight calculation formula is: $w_{pos} = \dfrac{e_{pos,pos=N,N-1,\ldots,1}}{\sum_{pos=1}^{N} e_{pos}}$ , in which $w_{pos}$ is the weight of a numbered position, $e_{pos}$ is the entropy value at this position, and N is the numbering annotation length of the longest sequence.

The humanized antibody sequence evaluation model was constructed, and expressed by the following formula:

$\text{Score}_{target} = \sum_{aa=1}^{N} w_{pos} p_{aa}$ , in which $Score_{target}$ represents the output evaluation value, $w_{pos}$ is the weight at a certain numbered position in the humanized antibody sequence, and $p_{aa}$ represents the probability of occurrence of an amino acid at this position, which is obtained by PSSM.

**Example 2 Effect of the humanized antibody sequence evaluation model**

Additional 4,999,335 human-derived antibody sequences (heavy chains), 4,936,514 murine-derived antibody sequences (heavy chains), 727,184 human-derived antibody sequences (light chains), and 727,184 murine-derived antibody sequences (light chains) were randomly extracted from the OAS database for testing to evaluate the ability of the above model to distinguish human-derived antibodies from murine-derived antibodies.

Additional 10,016,375 human-derived antibody sequences (heavy chains), 10,016,375 other species-derived antibody sequences (heavy chains), 2,026,539 human-derived antibody sequences (light chains), and 2,026,539 other species-derived antibody sequences (light chains) were randomly extracted from the OAS database for testing to evaluate the performance of the above model in distinguishing human-derived antibodies from other species-derived antibodies.

**The** above sequences were input into the humanized antibody sequence evaluation model constructed according to Example 1 to obtain a humanization evaluation value corresponding to each sequence, so as to evaluate the performance of the model in distinguishing human-derived antibodies from other species-derived antibodies.

A humanized antibody sequence coding method comprises the following steps:

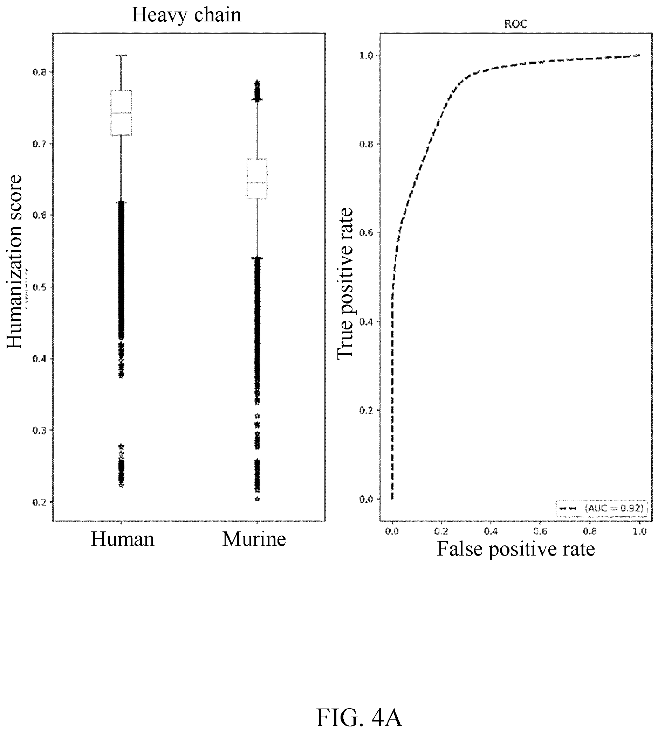

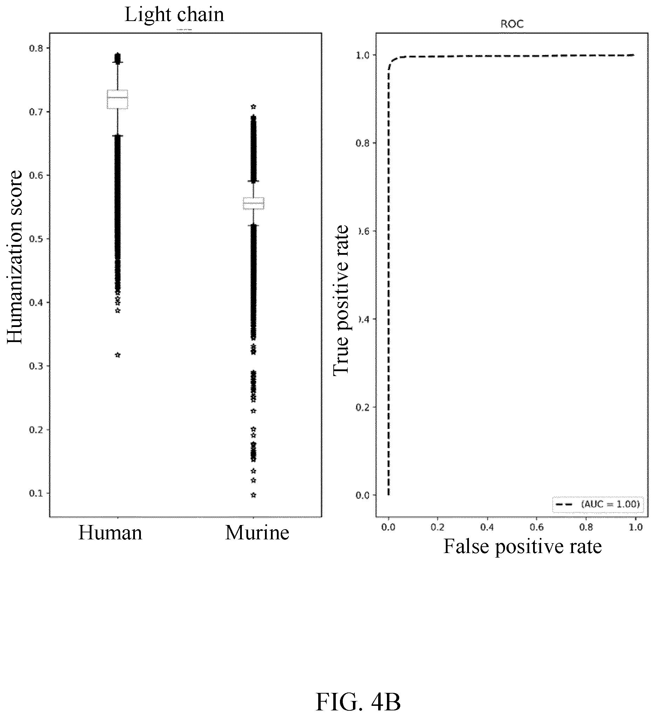

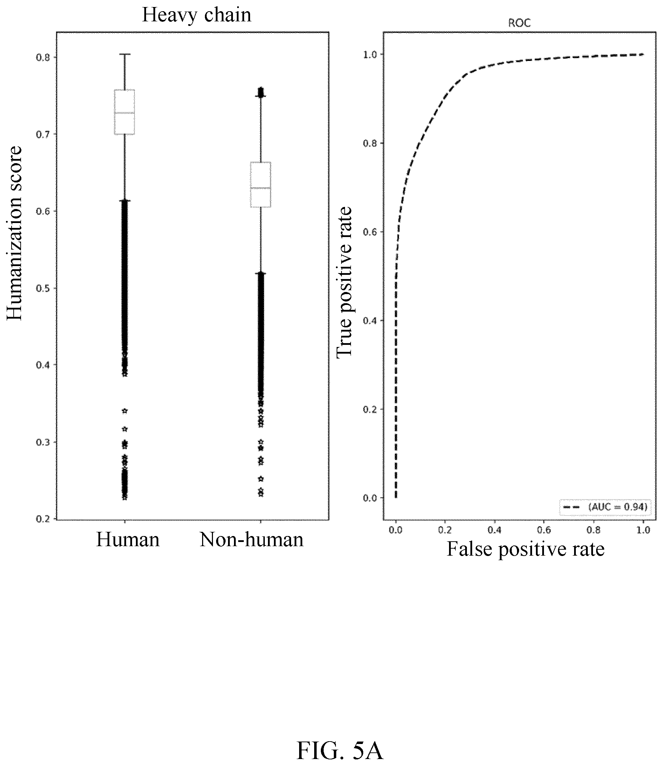

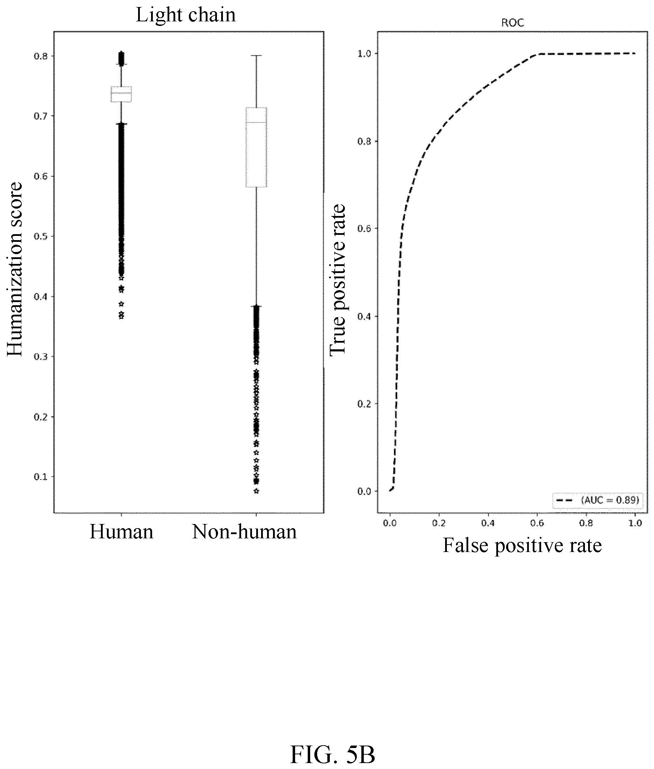

numbering and annotating the humanized antibody sequence, with the numbering annotation length being N;
according to the PSSM constructed in Example 1, inquiring the probability of occurrence of an amino acid at each position in the humanized antibody sequence;
according to the weight at each position and the probability of occurrence of an amino acid at each position as determined in Example 1, scoring the humanized antibody sequence according to the formula: $w_{pos} p_{aa}$ (namely weight $\times$ probability); and
processing according to classification (0 - for non-human and 1 - for human), plotting ROC curves (Receiver Operating Characteristic Curves) from the above results. The test results are as shown in FIGs. 4A-4B and FIGs. 5A-5B.

As shown in FIGs. 4A-4B and FIGs. 5A-5B, the humanized evaluation model constructed in the present application can distinguish the light chain and heavy chain sequences of human-/murine-derived antibodies, with the AUC values corresponding to the light and heavy chains being 1.00 and 0.92, respectively; and the humanized evaluation model can distinguish the light chain and heavy chain sequences of human-/other species-derived antibodies, with the AUC values corresponding to the light and heavy chains being 0.89 and 0.94, respectively. The test results indicate that the humanized antibody sequence evaluation model can effectively distinguish human-derived antibodies from murine-derived antibodies and human-derived antibodies from other species-derived antibodies, and is effective in the application of evaluating humanized antibody sequences.

Furthermore, after effective evaluation with the above constructed humanized antibody sequence evaluation model, 4-1BB agonist antibody (TNFRSF9 protein) is used as a specific example to evaluate rabbit-monoclonal-antibody-derived humanized antibody candidate sequences. According to the evaluation results, the top five ranked (N = 5) rabbit-monoclonal-antibody-derived humanized antibody light chain sequences and top five ranked (N = 5) rabbit-monoclonal-antibody-derived humanized antibody heavy chain sequences were given and subjected to biological experimental validation. Reference can be made to the following examples for details.

**Example 3 Determination of rabbit-monoclonal-antibody-derived humanized candidate sequences**

Specifically, the determination comprised the following steps:

A. Acquisition of an original rabbit monoclonal antibody:

1. Using TNFRSF9 protein as an antigen, a rabbit monoclonal antibody was prepared by subscribing to MonoRab™ rabbit monoclonal antibody customization service of Nanjing GenScript Biotech Co., Ltd. (https://www.genscript.com.cn/custom-rabbit-monoclonal-antibody-generation.html). The sequence of the antigen was:

MGNSCYNIVATLLLVLNFERTRSLQDPCSNCPAGTFCDNNRNQICSPCPPNSF

SSAGGQRTCDICRQCKGVFRTRKECSSTSNAECDCTPGFHCLGAGCSMCEQDCKQG

QELTKKGCKDCCFGTFNDQKRGICRPWTNCSLDGKSVLVNGTKERDVVCGPSPADLS

PGASSVTPPAPAREPGHSPQIISFFLALTSTALLFLLFFLTLRFSVVKRGRKKLLYIFKQP

FMRPVQTTQEEDGCSCRFPEEEEGGCEL (SEQ ID NO: 1).

2. The obtained rabbit monoclonal antibody was sequenced to obtain the amino acid sequences of the light and heavy chain variable regions of the rabbit monoclonal antibody. Taking clone 81E11 as an example, the complete variable region sequences of the light and heavy chains were respectively as follows:

The complete variable region of the light chain of the rabbit monoclonal antibody:

AAVLTQTPSPVSVTVGGTVTINCQASQSVDNNNYLAWFQQKPGQPPKQLIYSA

STLASGVSSRFKGSGSGTQFTLTISGVQCDDAATYYCLGEFSASSGDWNAFGGGTEV

VVK (SEQ ID NO: 2).

The complete variable region of the heavy chain of the rabbit monoclonal antibody:

QSVKESEGGLFKPTDTLTLACTVSGFSLSYNAITWVRQAPGNGLEWIGVINYDG

TTVYASWAKSRSTITRNTNLNTVTLKMTSLTAADTATYFCARNFNIWGPGTLVTVSS

(SEQ ID NO: 3).

B. Humanization of the rabbit monoclonal antibody:

1. An optimal human-derived template was sought:
1.1 Taking the complete variable region sequence (SEQ ID NO: 2) of the light chain of the rabbit monoclonal antibody as obtained in step A as an input, a search was performed in the OAS (Observed Antibody Space) database by BLAST tool to obtain the human-derived antibody sequence with the highest sequence similarity, which was used as the template sequence of the light chain. The human-derived antibody light chain template sequence was:

DIQMTQSPSSLSASVGDTVTITCRASQSISTYLSWFQQKPGKAPKLLIYVASSLQ SGVPSRFSGSGSGTEFTLTIAGLQLDDLATYYCQQYNSFELSFGGGTKVDIK (SEQ ID NO: 4).

1.2 Taking the complete variable region sequence (SEQ ID NO: 3) of the heavy chain of the rabbit monoclonal antibody as obtained in step A as an input, a search was performed in the Observed Antibody Space (OAS) database by BLAST tool to obtain the human-derived antibody sequence with the highest sequence similarity, which was used as the template sequence of the heavy chain. The heavy chain template sequence of the human-derived antibody was:

QVQLQESGPGLVKPSETLSLTCTVSGGSIDTYYWSWIRQPPGKGLEWIGYLYN PSLKSRATISLDTSKNQISLKMRSMTAADTAMYFCARDPNRAAAGAFDIWGPGTMVTV SS (SEQ ID NO: 5).

2. Using ANARCI unified numbering system, the complete variable region sequence (SEQ ID NO: 2) of the light chain of the rabbit monoclonal antibody, the complete variable region sequence (SEQ ID NO: 3) of the heavy chain of the rabbit monoclonal antibody, and the human-derived antibody light chain template (SEQ ID NO: 4) and the human-derived antibody heavy chain template (SEQ ID NO: 5) obtained in step 1 were numbered specifically using Chothia numbering scheme. Exemplary numbered sequences were as shown below.

Numbered complete variable region sequence of the light chain of the rabbit monoclonal antibody:

1,2,3,4,5,6,7,8,9,10,11,12,13,14,15,16,17,18,19,20,21,22,23,24,25,26,27,28,29,3 0,30A,30B,31,32,33,34,35,36,37,38,39,40,41,42,43,44,45,46,47,48,49,50,51,52,53,54,5 5,56,57,58,59,60,61,62,63,64,65,66,67,68,69,70,71,72,73,74,75,76,77,78,79,80,81,82,8 3,84,85,86,87,88,89,90,91,92,93,94,95,95A,95B,95C,95D,96,97,98,99,100,101,102,103 ,104,105,106,107. A,A,V,L,T,Q,T,P,S,P,V,S,V,T,V,G,G,T,V,T,I,N,C,Q,A,S,Q,S,V,D,N,N,N,Y,L,A,W,F ,Q,Q,K,P,G,Q,P,P,K,Q,L,I,Y,S,A,S,- T,L,A,S,G,V,S,S,R,F,K,G,S,G,S,G,T,Q,F,T,L,T,I,S,G, V,Q,C,D,D,A,A,T,Y,Y,C,L,G,E,F,S,A,S,S,G,D,W,N,A,F,G,G,G,T,E,V,V,V,K.

Numbered complete variable region sequence of the heavy chain of the rabbit monoclonal antibody:

1,2,3,4,5,6,7,8,9,10,11,12,13,14,15,16,17,18,19,20,21,22,23,24,25,26,27,28,29,3 0,31,32,33,34,35,36,37,38,39,40,41,42,43,44,45,46,47,48,49,50,51,52,53,54,55,56,57,5 8,59,60,61,62,63,64,65,66,67,68,69,70,71,72,73,74,75,76,77,78,79,80,81,82,82A,82B,8 2C,83,84,85,86,87,88,89,90,91,92,93,94,95,96,97,98,99,100,101,102,103,104,105,106, 107,108,109,110,111,112,113. Q,-,S,V,K,E,S,E,G,G,L,F,K,P,T,D,T,L,T,L,A,C,T,V,S,g,f,s,I,s,Y,N,A,I,T,W,V,R,Q,A ,P,G,N,G,L,E,W,I,G,V,I,N,Y,D,G,T,- T,V,Y,A,S,W,A,K,S,R,S,T,I,T,R,N,T,N,L,N,T,V,T,L,K, M,T,S,L,T,A,A,D,T,A,T,Y,F,C,A,R,N,F,-,-,-,-,N,I,W,G,P,G,T,L,V,T,V,S,S.

Numbered human-derived antibody light chain template sequence:

1,2,3,4,5,6,7,8,9,10,11,12,13,14,15,16,17,18,19,20,21,22,23,24,25,26,27,28,29,3 0,31,32,33,34,35,36,37,38,39,40,41,42,43,44,45,46,47,48,49,50,51,52,53,54,55,56,57,5 8,59,60,61,62,63,64,65,66,67,68,69,70,71,72,73,74,75,76,77,78,79,80,81,82,83,84,85,8 6,87,88,89,90,91,92,93,94,95,96,97,98,99,100,101,102,103,104,105,106,107. D,I,Q,M,T,Q,S,P,S,S,L,S,A,S,V,G,D,T,V,T,I,T,C,R,A,S,Q,S,I,S,T,Y,L,S,W,F,Q,Q, K,P,G,K,A,P,K,L,L,I,Y,V,A,S,S,L,Q,S,G,V,P,S,R,F,S,G,S,G,S,G,T,E,F,T,L,T,I,A,G,L,Q,L, D,D,L,A,T,Y,Y,C,Q,Q,Y,N,S,F,E,L,S,F,G,G,G,T,K,V,D,I,K.

Numbered human-derived antibody heavy chain template sequence:

1,2,3,4,5,6,7,8,9,10,11,12,13,14,15,16,17,18,19,20,21,22,23,24,25,26,27,28,29,3 0,31,32,33,34,35,36,37,38,39,40,41,42,43,44,45,46,47,48,49,50,51,52,53,54,55,56,57,5 8,59,60,61,62,63,64,65,66,67,68,69,70,71,72,73,74,75,76,77,78,79,80,81,82,82A,82B,8 2C,83,84,85,86,87,88,89,90,91,92,93,94,95,96,97,98,99,100,100A,100B,100C,100D,10 1,102,103,104,105,106,107,108,109,110,111,112,113. Q,V,Q,L,Q,E,S,G,P,G,L,V,K,P,S,E,T,L,S,L,T,C,T,V,S,G,G,S,I,D,T,Y,Y,W,S,W,I,R, Q,P,P,G,K,G,L,E,W,I,G,-,-,-,-,-,-,Y,L,-,Y,N,P,S,L,K,S,R,A,T,I,S,L,D,T,S,K,N,Q,I,S,L,K,M, R,S,M,T,A,A,D,T,A,M,Y,F,C,A,R,D,P,N,R,A,A,A,G,A,F,D,I,W,G,P,G,T,M,V,T,V,S,S.

In the above numbered sequences, for example, taking the numbered complete variable region sequence of the light chain of the rabbit monoclonal antibody as an example, 1 corresponds to A; and taking the numbered complete variable region sequence of the heavy chain of the rabbit monoclonal antibody as an example, "-" represents a gap position.

**3.** The human-derived antibody light chain and heavy chain template sequences were subjected to CDR region substitution, which comprised the following steps:

3.1 The rabbit monoclonal antibody light chain and heavy chain and the human-derived antibody light chain template and heavy chain template sequences obtained in step 1 were aligned to the same coordinate system, and the gap positions were filled with "-".

3.2 According to the numbering in step 2, the CDR regions of the light chain and heavy chain of the rabbit monoclonal antibody and the light chain template and heavy chain template of the human-derived antibody obtained in step 1 were respectively labeled. The sequences of these CDR regions were:

heavy chain CDR-H1 of the rabbit monoclonal antibody: GFSLSYN (SEQ ID NO: 6);
heavy chain CDR-H2 of the rabbit monoclonal antibody: NYDGT (SEQ ID NO: 7);
heavy chain CDR-H3 of the rabbit monoclonal antibody: FN (SEQ ID NO: 8);
light chain CDR-L1 of the rabbit monoclonal antibody: SQSVDNNNY (SEQ ID NO: 9);
light chain CDR-L2 of the rabbit monoclonal antibody: SAS (SEQ ID NO: 10);
light chain CDR-L3 of the rabbit monoclonal antibody: EFSASSGDWN (SEQ ID NO: 11);
human-derived antibody heavy chain template CDR-H1: GGSIDTY (SEQ ID NO: 12);
human-derived antibody heavy chain template CDR-H2: Y (SEQ ID NO: 13);
human-derived antibody heavy chain template CDR-H3: PNRAAAGAFD (SEQ ID NO: 14);
human-derived antibody light chain template CDR-L1: SQSISTY (SEQ ID NO: 15);
human-derived antibody light chain template CDR-L2: VAS (SEQ ID NO: 16); and
human-derived antibody light chain template CDR-L3: YNSFEL (SEQ ID NO: 17).

3.3 The CDR regions of the light and heavy chains of the rabbit monoclonal antibody were respectively substituted for the corresponding CDR regions of the light and heavy chain templates of the human-derived antibody in step 3.2; and the light and heavy chain template sequences of the humanized antibody after the substitution were respectively as follows:

the humanized antibody light chain template sequence after the substitution:

DIQMTQSPSSLSASVGDTVTITCRA**SQSVDNNNY**LSWFQQKPGKAPKLLIY**SAS** SLQSGVPSRFSGSGSGTEFTLTIAGLQLDDLATYYCQQ**EFSASSGDWN**SFGGGTKVD IK (SEQ ID NO: 18).

**the** humanized antibody heavy chain template sequence after the substitution:

QVQLQESGPGLVKPSETLSLTCTVS**GFSLSYN**YWSWIRQPPGKGLEWIG**NYD GT**LYNPSLKSRATISLDTSKNQISLKMRSMTAADTAMYFCARD**FN**IWGPGTMVTVSS (SEQ ID NO: 19).

In the sequences, the bold parts represent the substituted CDR regions.

**4.** Cysteine (CYS) treatment was performed, comprising the following steps:

4.1 The three-dimensional structures of light and heavy chains of the rabbit monoclonal antibody were respectively predicted by using AlphaFold II.

4.2 According to the predicted three-dimensional structures of the light and heavy chains of the rabbit monoclonal antibody, the distance between all pairs of CYS in the rabbit monoclonal antibody sequence was calculated. As for the pairs of CYS with a distance of 4 to 7 angstroms, if there was no corresponding amino acid in the human-derived antibody template after the substitution, serine (SER) was inserted at the positions, and the pairs of CYS in the CDR regions were not substituted.

4.3 On the basis of step 4.2, a single CYS was substituted. Where there was a single CYS in the rabbit monoclonal antibody sequence, Ser was inserted if there was no amino acid at the corresponding position of the human-derived antibody template after the substitution. The light and heavy chains after CYS substitution were as shown below. In this example, the light and heavy chain sequences were not subjected to the substitution.

The light chain sequence after CYS substitution:

DIQMTQSPSSLSASVGDTVTITCRA**SQSVDNNNY**LSWFQQKPGKAPKLLIY**SAS** SLQSGVPSRFSGSGSGTEFTLTIAGLQLDDLATYYCQQ**EFSASSGDWN**SFGGGTKVD IK (SEQ ID NO: 20).

The heavy chain sequence after CYS substitution:

QVQLQESGPGLVKPSETLSLTCTVS**GFSLSYN**YWSWIRQPPGKGLEWIG**NYD GT**LYNPSLKSRATISLDTSKNQISLKMRSMTAADTAMYFCARD**FN**IWGPGTMVTVSS (SEQ ID NO: 21).

**5.** Light chain E-F ring treatment was performed, comprising the following steps:
5.1 A search was performed in UniRef90 database for the human-derived antibody light chain template sequence by using BLAST to obtain a light chain Multiple Sequence Alignment (MSA) result.

5.2 According to the MSA obtained in step 5.1, a Position Specific Scoring Matrix (PSSM) for light chain E-F rings was constructed.

5.3 According to the PSSM obtained in step 5.2, several candidate E-F ring sequences were randomly generated according to the probability of occurrence of an amino acid corresponding to each position. The candidate E-F ring sequences were (taking 10 sequences as examples):

SSLQPED (SEQ ID NO: 22), NSLQPED (SEQ ID NO: 23), TSLQPED (SEQ ID NO: 24), SNLQPED (SEQ ID NO: 25), and NNLQPED (SEQ ID NO: 26).
TNLQPED (SEQ ID NO: 27), SGLQPED (SEQ ID NO: 28), NGLQPED (SEQ ID NO: 29), TGLQPED (SEQ ID NO: 30), and SSMQPED (SEQ ID NO: 31).

5.4 The candidate E-F ring sequences generated in step 5.3 were successively substituted for the corresponding sequence in the light chain sequence (SEQ ID NO: 20) obtained in step 4.3 to generate several candidate sequences. Taking substitution with SSLQPED (SEQ ID NO: 22) as an example, one candidate sequence was listed schematically:

DIQMTQSPSSLSASVGDTVTITCRA**SQSVDNNNY**LSWFQQKPGKAPKLLIY**SAS** SLQSGVPSRFSGSGSGTEFTLTIASSLQPEDATYYCQQ**EFSASSGDWN**SFGGGTKVD IK (SEQ ID NO: 44).

6. Heavy chain D-E ring treatment was performed, comprising the following steps:
6.1 A search was performed in the UniRef90 database for the human-derived antibody heavy chain template sequence by BLAST to obtain a heavy chain MSA result.

6.2 According to the MSA obtained in step 6.1, a PSSM for heavy chain D-E rings was constructed.

6.3 According to the PSSM obtained in step 6.2, several candidate D-E ring sequences were randomly generated according to the probability of occurrence of an amino acid corresponding to each position. The candidate D-E ring sequences were (taking 10 sequences as examples):

VDTSKN (SEQ ID NO: 32), IDTSKN (SEQ ID NO: 33), LDTSKN (SEQ ID NO: 34), VETSKN (SEQ ID NO: 35), and IETSKN (SEQ ID NO: 36).

LETSKN (SEQ ID NO: 37), VNTSKN (SEQ ID NO: 38), INTSKN (SEQ ID NO: 39), LNTSKN (SEQ ID NO: 40), and VDMSKN (SEQ ID NO: 41).

6.4 The candidate D-E ring sequences generated in step 6.3 were successively substituted for the corresponding sequence in the heavy chain sequence (SEQ ID NO: 21) obtained in step 4.3 to generate several candidate sequences. Taking substitution with VDTSKN (SEQ ID NO: 32) as an example, one candidate sequence was listed schematically:

QVQLQESGPGLVKPSETLSLTCTVS**GFSLSYN**YWSWIRQPPGKGLEWIG**NYD GT**LYNPSLKSRATISLVDTSKNISLKMRSMTAADTAMYFCARD**FN**IWGPGTMVTVSS (SEQ ID NO: 45).

7. Back mutation was performed, comprising the following steps:

7.1 A search was performed in the UniRef90 database for the light and heavy chain variable region sequences (SEQ ID NO: 2 and SEQ ID NO: 3) of the parent rabbit monoclonal antibody, respectively, by BLAST to obtain light and heavy chain MSA results, and according to the MSA results, the corresponding PSSM was obtained.

7.2 From the PSSM obtained in step 7.1, highly conserved sites in the light and heavy chain variable regions were obtained. These sites were respectively:

in the heavy chain: 13P, 17L, 19L, and 21C;
in the light chain: 1A, 5T, 6Q, 16G, and 23C.

7.3 It was determined at the sites obtained in step 7.2 whether the amino acids at the corresponding positions of the several candidate sequences obtained in steps 5.4 and 6.4 were consistent with those in the parent rabbit monoclonal antibody sequence. If not consistent, they were substituted back with the amino acids at the corresponding positions of the parent rabbit monoclonal antibody sequence. At this time, the light and heavy chain variable region sequences were (taking one candidate antibody as an example):

**the** light chain variable region sequence after back mutation:

<u>A</u>IQMTQSPSSLSASVGDTVTITCRA**SQSVDNNNY**LSWFQQKPGKAPKLLIY**SAS** SLQSGVPSRFSGSGSGTEFTLTIASSLQPEDATYYCQQ**EFSASSGDWN**SFGGGTKVD IK (SEQ ID NO: 42);

**the** heavy chain variable region sequence after back mutation:

QVQLQESGPGLVKPSETLSLTCTVS**GFSLSYN**YWSWIRQPPGKGLEWIG**NYD GT**LYNPSLKSRATISLVDTSKNISLKMRSMTAADTAMYFCARD**FN**IWGPGTMVTVSS (SEQ ID NO: 43).

**The** underlined parts represent the amino acids substituted back by back mutation, and the first site of the light chain underwent back mutation, while the heavy chain was not modified.

**Example 4 Evaluation of candidate sequences by the humanized antibody sequence evaluation model**

**The** evaluation of candidate sequences by the humanized antibody sequence evaluation model comprised the following steps:

All the candidate humanized antibody sequences to be evaluated finally obtained in Example 3 were subjected to sequence coding according to the humanized antibody sequence coding method in Example 2.

According to the humanized antibody sequence evaluation model constructed in Example 1, all the candidate humanized antibody sequences to be evaluated were scored: $Score_{target} = \sum_{aa=1}^{N} w_{pos} p_{aa}$, in which the meaning of each symbol was completely consistent with that described in Example 1.

All the scored candidate humanized antibody sequences were ranked according to the scores $Score_{target}$ from high to low.

The top five light chains and the top five heavy chains with the highest scores were selected respectively. The scores were as shown in Table 1:

Table 1

| Chain No. | Humanization score |
|---|---|
| Heavy chain 1 (H1) | 0.63768292 |
| Heavy chain 2 (H2) | 0.615641904 |
| Heavy chain 3 (H3) | 0.615631875 |
| Heavy chain 4 (H4) | 0.615630353 |
| Heavy chain 5 (H5) | 0.615612174 |
| Light chain 1 (L1) | 0.588197557 |
| Light chain 2 (L2) | 0.568877437 |
| Light chain 3 (L3) | 0.568866752 |
| Light chain 4 (L4) | 0.568859278 |
| Light chain 5 (L5) | 0.568858668 |

**Example 5 Computational simulation and biological validation of antibody-antigen binding**

1. Computational simulation of antibody-antigen binding was performed, comprising the following steps:

1.1 The top five light chains and five heavy chains with the highest scores obtained in Example 4 were paired in pairs.
1.2 The antibody structures after pairing in step 1.1 were predicted by AbodyBuilder.
1.3 The antigen structure was predicted by AlphaFold II.
1.4 All the antibody structures predicted in step 1.2 were respectively subjected to simulation of binding with the antigen structure predicted in step 1.3 by ZDOCK.
1.5 The simulation results in step 1.4 were ranked, and the top three combinations ranked as the best were selected.

2. The three antibodies in step 1.5 were subjected to biological validation and named 81E11H3L1, 81E11H1L1, and 81E11H4L1, respectively. The process specifically comprised the following steps:

2.1 A signal peptide was added to both the heavy chain variable region and light chain variable region of the candidate antibody at the N-terminus. The sequence of the signal peptide was: MGWSCIILFLVATATGVHS (SEQ ID NO: 46).
2.2 A constant region sequence was added to the heavy chain variable region at the C-terminus to form a complete heavy chain. The constant region sequence was:

ASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKVEPKSCDKTHTCPP
CPAPELLGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPAPIEKTISKAKGQPR
EPQVYTLPPSRDELTKNQVSLTCLVKGFYPSDIAVEWESNGQPENNYKTTPPVLDSDG
SFFLYSKLTVDKSRWQQGNVFSCSVMHEALHNHYTQKSLSLSPGK (SEQ ID NO: 47).

**2.3** A constant region sequence was added to the light chain variable region at the C-terminus to form a complete light chain. The constant region sequence was:

RTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC (SEQ ID NO: 48).

**Example 6 Validation of a humanized antibody of 4-1BB agonist antibody**

**The** experimental methods in the following examples are all conventional methods, unless otherwise specified. The experimental materials used in the following examples were all purchased from conventional biochemical reagent companies, unless otherwise specified. The quantitative experiments in the following examples were performed in triplicate, and the results were averaged.

In the present application, the 4-1BB agonist antibody was used. 31 positive clones were obtained by immunizing a rabbit. Taking clone 81E11 as an example, a total of 25 humanized antibodies were obtained through the above implementation steps. The obtained humanized antibody was subjected to computational simulation of antibody-antigen binding, and the top three antibodies ranked as the best were selected for expression, followed by an ELISA binding experiment to validate the positive binding to the antigen.

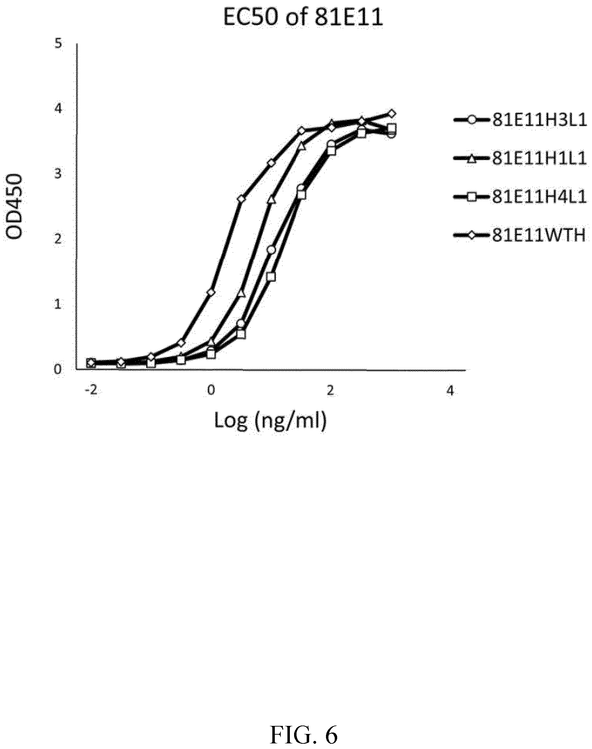

ELISA detection of antibody affinity: An order for the whole antibody sequence was placed with the Antibody Department of Nanjing GenScript Biotech Co., Ltd. for the complete synthesis of the antibody and the detection of antigen-antibody affinity. The antibody affinity detection method was an enzyme-linked immunosorbent assay (ELISA) based on an indirect method. Indirect ELISA was used for evaluating the ability of the purified antibody to bind the antigen TNFRSF9 protein. The specific steps were as follows: the ELISA plate was coated with 0.5 $\mu$g/ml recombinant TNFRSF9 protein in PBS at 100 $\mu$l/well at 4°C overnight. The plate was washed with PBS-T (0.05% Tween) and blocked with 250 $\mu$l/well of PBST containing 1% BSA at 37°C for 2 hours. Subsequently, the blocking solution was discarded, and 100 $\mu$l of the 1 $\mu$g/ml purified antibody was added to the first well and 3-fold diluted in a gradient to prepare a total of 11 serial test concentrations, and additionally a blank well was prepared. The plate was then incubated at 37°C for 1 hour. The plate was washed three times with PBST and incubated with 100 $\mu$l/well of horseradish peroxidase-conjugated goat anti-mouse IgG (Fc-specific) secondary antibody (Jackson, 115-035-071) at 37°C for 0.5 hours. The plate was washed four times with PBST, a TMB color development solution (GenScript) was then added, and the plate was incubated in the dark at 25°C for 15 minutes. The reaction was terminated by adding 50 $\mu$l of a 1M HCl termination solution (Sinopharm, 10011018). The plate was read at 450 nm using a microplate reader. The results of the ELISA binding experiment were as shown in FIG. 6. After experimental validation, a humanized antibody with positive binding was obtained, which indicated the effectiveness of the antibody humanization method disclosed in the present application.

The EC50 values were as shown in Table 2.

Table 2

| Affinity ranking | Clone name | EC50 (ng/ml) |
|---|---|---|
| 1 | 81E11WTH | 2.598 |
| 2 | 81E11H1L1 | 7.389 |
| 3 | 81E11H3L1 | 13.830 |
| 4 | 81E11H4L1 | 18.640 |

The results in Table 2 indicate that compared with the control 81E11WTH, the three humanized antibodies, 81E11H1L1, 81E11H3L1, and 81E11H4L1, have better binding activities, and the affinity loss is within an acceptable range.

A method for constructing a humanized antibody sequence evaluation model, a method for obtaining a candidate humanized antibody sequence by means of evaluation, and a method for humanizing a monoclonal antibody disclosed in the present application bring about beneficial effects including but not limited to the following aspects: (1) The humanized antibody sequence evaluation model constructed in the present application can evaluate a plurality of candidate humanized sequences involved and finally give an effective humanized antibody. This model is suitable for the evaluation of the humanization of antibodies derived from all species, and has lower requirements for computing resources, low cost, and less time consumption. (2) In the method for obtaining a candidate humanized antibody sequence by means of evaluation as provided by the present application, the humanized antibody sequence evaluation model constructed based on the entropy values is used, the sequences to be evaluated can be scored to screen out effective humanized antibody sequences. (3) By the method for humanizing a monoclonal antibody as provided by the present application, humanized antibodies with positive antigen binding are obtained, with the affinity loss after humanization being in an acceptable range, and the humanized antibodies are effective and can be used for diagnosis and detection, antibody imaging, and treatment of diseases sensitive to a monoclonal antibody-based therapy. It should be noted that different embodiments may have different beneficial effects, and the beneficial effects that may be produced in the different embodiments may be any one or a combination of the above, or may be any other beneficial effects that may be obtained.

It should be understood by those skilled in the art that the above embodiments are only for illustrating the present application and do not limit the present application. Any modifications, equivalent substitutions, alterations, and the like made within the spirit and principle of the present application should fall within the scope of protection of the present application.

**Claims**

1. A method for constructing a humanized antibody sequence evaluation model, **characterized in that** the method comprises:

   acquiring amino acid sequences of a plurality of human-derived antibody templates and numbering the amino acid sequences;
   calculating an entropy value at each numbered position; and
   constructing a humanized antibody sequence evaluation model on the basis of the entropy value.

2. The method according to claim 1, **characterized in that** the entropy value is determined by a position specific scoring matrix, wherein the method further comprises:

   according to the numbered longest sequence, determining a multiple sequence alignment result of the amino acid sequences of the plurality of human-derived antibody templates, wherein gap positions are filled by inserting symbols; and
   on the basis of the multiple sequence alignment result, constructing the position specific scoring matrix of the amino acid sequences of the plurality of human-derived antibody templates.

3. The method according to claim 1 or 2, **characterized in that** the entropy value is calculated by the following formula:

$$\frac{1}{n}\sum_{i=1}^{n} p_i log p_i,$$

   in which n is the total number of the types of all amino acids and inserted symbols appearing at a certain numbered position and is maximally 21, i is an index of n, and $p_i$ is the probability of occurrence of the ith amino acid as derived from the position specific scoring matrix at this position.

4. The method according to any one of claims 1-3, **characterized in that** constructing a humanized antibody sequence evaluation model on the basis of the entropy value comprises:
   determining a weight at each numbered position, wherein the weight is negatively correlated with the entropy value.

5. The method according to claim 4, **characterized in that** the weight is calculated by the following formula:

$$w_{pos} = \frac{e_{pos,pos=N,N-1,...,1}}{\sum_{pos=1}^{N} e_{pos}},$$

in which $w_{pos}$ is the weight of a numbered position, $e_{pos}$ is the entropy value at this position, and N is the numbering annotation length of the longest sequence.

6. The method according to claim 5, **characterized in that** the humanized antibody sequence evaluation model is expressed by the following formula:

$$\text{Score}_{target} = \sum_{aa=1}^{N} w_{pos} p_{aa},$$

in which $Score_{target}$ represents the output evaluation value, $w_{pos}$ is the weight at a certain numbered position in the humanized antibody sequence, and $p_{aa}$ represents the probability of occurrence of an amino acid at this position.

7. The method according to any one of claims 1-6, **characterized in that** the humanized antibody sequence evaluation model is used for evaluating humanized sequences of antibodies derived from all species.

8. A method for obtaining a candidate humanized antibody sequence by means of evaluation, **characterized in that** the method comprises:

numbering a humanized antibody sequence to be evaluated;
determining the weight at each numbered position in the humanized antibody sequence to be evaluated and the probability of occurrence of an amino acid at this numbered position;
on the basis of the weight and the probability of occurrence of the amino acid, evaluating the humanized antibody sequence to be evaluated using the humanized antibody sequence evaluation model constructed by the method according to claim 6 to obtain an evaluation value; and
if the evaluation value meets a preset condition, determining the humanized antibody sequence to be evaluated corresponding to the evaluation value as a candidate humanized antibody sequence.

9. The method according to claim 8, **characterized in that** the preset condition is that the evaluation value exceeds a preset threshold or is ranked higher than a certain value after ranking.

10. The method according to claim 8 or 9, **characterized by** further comprising determining a humanized antibody sequence of interest by:

predicting an antibody structure of the candidate humanized antibody sequence;
simulating the binding of the candidate antibody structure with a corresponding antigen to obtain the candidate antibody structure;
selecting the candidate antibody structure for biological experimental validation; and
determining the humanized antibody sequence of interest based on the result of the biological experimental validation.

11. A method for humanizing a monoclonal antibody, **characterized in that** the method comprises:

determining a human-derived antibody light chain template sequence and a human-derived antibody heavy chain template sequence for the monoclonal antibody respectively;
substituting CDR regions in a light chain and heavy chain of the monoclonal antibody for corresponding CDR regions in the human-derived antibody light chain template sequence and the human-derived antibody heavy chain template sequence respectively, to obtain a humanized antibody light chain template sequence and humanized antibody heavy chain template sequence resulting from the CDR region substitution;
subjecting the humanized antibody light chain template sequence resulting from the CDR region substitution to an E-F ring treatment to obtain a plurality of candidate humanized antibody light chain sequences; and
subjecting the humanized antibody heavy chain template sequence resulting from the CDR region substitution to a D-E ring treatment to obtain a plurality of candidate humanized antibody heavy chain sequences.

12. The method according to claim 11, **characterized in that** subjecting the humanized antibody light chain template sequence resulting from the CDR region substitution to an E-F ring treatment to obtain a plurality of candidate humanized antibody light chain sequences comprises:

> performing a search in a database for the humanized antibody light chain template sequences resulting from the CDR region substitution to obtain a multiple sequence alignment result;
> according to the multiple sequence alignment result, constructing a position specific scoring matrix for E-F rings;
> generating a plurality of E-F ring sequences according to the position specific scoring matrix for E-F rings; and
> substituting the plurality of E-F ring sequences for the corresponding sequence in the humanized antibody light chain template sequence resulting from the CDR region substitution to obtain the plurality of candidate humanized antibody light chain sequences.

13. The method according to claim 11, **characterized in that** subjecting the humanized antibody heavy chain template sequence resulting from the CDR region substitution to a D-E ring treatment to obtain a plurality of candidate humanized antibody heavy chain sequences comprises:

> performing a search in a database for the humanized antibody heavy chain template sequences resulting from the CDR region substitution to obtain a multiple sequence alignment result;
> according to the multiple sequence alignment result, constructing a position specific scoring matrix for D-E rings;
> generating a plurality of D-E ring sequences according to the position specific scoring matrix for D-E rings; and
> substituting the plurality of D-E ring sequences for the corresponding sequence in the humanized antibody heavy chain template sequence resulting from the CDR region substitution to obtain the plurality of candidate humanized antibody heavy chain sequences.

14. The method according to any one of claims 11-13, **characterized in that** the method further comprises:
subjecting highly conserved sites in the plurality of candidate humanized antibody light chain sequences and the plurality of candidate humanized antibody heavy chain sequences respectively to back mutation.

15. The method according to claim 14, **characterized in that** subjecting highly conserved sites in the plurality of candidate humanized antibody light chain sequences and the plurality of candidate humanized antibody heavy chain sequences respectively to back mutation comprises:

> determining highly conserved sites in the light chain and heavy chain sequences of the monoclonal antibody respectively;
> determining whether the plurality of candidate humanized antibody light chain sequences and the plurality of candidate humanized antibody heavy chain sequences are respectively consistent with the amino acids of the light chain and heavy chain of the monoclonal antibody at the highly conserved sites; and
> if not consistent, substituting the amino acids at the highly conserved sites in the plurality of candidate humanized antibody light chain sequences and the plurality of candidate humanized antibody heavy chain sequences back with the amino acids at the corresponding positions in the light chain and heavy chain of the monoclonal antibody.

16. **The** method according to any one of claims 11-15, **characterized in that** the monoclonal antibody is a rabbit monoclonal antibody, and the method further comprises:

> predicting three-dimensional structures of a light chain and heavy chain of the rabbit monoclonal antibody;
> according to the predicted three-dimensional structures, if there are a pair of cysteines in the rabbit monoclonal antibody, with the distance between this pair of cysteines being between 4 and 7 angstroms, and no amino acid is present at the corresponding positions in the humanized antibody light chain template sequence and humanized antibody heavy chain template sequence resulting from the CDR region substitution, inserting serine at the positions, with the positions excluding the CDR region; and
> if there is only a single cysteine in the rabbit monoclonal antibody and no amino acid is present at the corresponding position in the humanized antibody light chain template sequence and humanized antibody heavy chain template sequence resulting from the CDR region substitution, inserting serine at the position.

17. **The** method according to any one of claims 11-16, **characterized in that** the method further comprises:
evaluating the light chain sequences and heavy chain sequences of the candidate humanized antibodies respectively by the humanized antibody sequence evaluation model constructed using the method according to claim 6 to obtain light chain sequences and heavy chain sequences of humanized antibodies.

**18.** The method according to claim 17, **characterized in that** the method further comprises:
subjecting the humanized antibodies to biological experimental validation to determine the humanized antibody sequence of interest.

Determining a human-derived antibody light chain template sequence and a human-derived antibody heavy chain template sequence for the monoclonal antibody respectively — 101

Substituting CDR regions in a light chain and heavy chain of the monoclonal antibody for corresponding CDR regions in the human-derived antibody light chain template sequence and the human-derived antibody heavy chain template sequence respectively, to obtain a humanized antibody light chain template sequence and a humanized antibody heavy chain template sequence resulting from the CDR region substitution — 102

Subjecting the humanized antibody light chain template sequence resulting from the CDR region substitution to an E-F ring treatment to obtain a plurality of candidate humanized antibody light chain sequences — 103

Subjecting the humanized antibody heavy chain template sequence resulting from the CDR region substitution to a D-E ring treatment to obtain a plurality of candidate humanized antibody heavy chain sequences — 104

Subjecting highly conserved sites in the plurality of candidate humanized antibody light chain sequences and the plurality of candidate humanized antibody heavy chain sequences respectively to back mutation — 105

FIG. 1

FIG. 2

Numbering a humanized antibody sequence to be evaluated — 301

Determining the weight at each numbered position in the humanized antibody sequence to be evaluated and the probability of occurrence of an amino acid at this numbered position — 302

On the basis of the weight and the probability of occurrence of the amino acid, evaluating the humanized antibody sequence to be evaluated using the humanized antibody sequence evaluation model determined above to obtain an evaluation value — 303

If the evaluation value meets a preset condition, determining the humanized antibody sequence to be evaluated corresponding to the evaluation value as a candidate humanized antibody sequence — 304

FIG. 3

FIG. 4A

FIG. 4B

FIG. 5A

FIG. 5B

FIG. 6

## INTERNATIONAL SEARCH REPORT

| International application No. |
| --- |
| **PCT/CN2023/127070** |

### A.    CLASSIFICATION OF SUBJECT MATTER

G16B 30/10(2019.01)i

According to International Patent Classification (IPC) or to both national classification and IPC

### B.    FIELDS SEARCHED

Minimum documentation searched (classification system followed by classification symbols)

IPC：G16B

Documentation searched other than minimum documentation to the extent that such documents are included in the fields searched

Electronic data base consulted during the international search (name of data base and, where practicable, search terms used)

CNTXT; CNABS; CNKI; VEN; USTXT; WOTXT; EPTXT: 人源化, 抗体, 评估, 模型, 编号, 熵值, 权重, 单克隆, 轻链, 重链, CDR, 替换, humanization, antibody, evaluation, model, number, entropy, weight, monoclonal, light chain, heavy chain, CDR, replacement

### C.    DOCUMENTS CONSIDERED TO BE RELEVANT

| Category* | Citation of document, with indication, where appropriate, of the relevant passages | Relevant to claim No. |
| --- | --- | --- |
| X | CN 1839144 A (EPITOMICS, INC.) 27 September 2006 (2006-09-27) description, pages 4-37 | 11-16 |
| A | CN 1839144 A (EPITOMICS, INC.) 27 September 2006 (2006-09-27) entire document | 1-10, 17, 18 |
| A | CN 103265631 A (THE FOURTH MILITARY MEDICAL UNIVERSITY OF THE CHINESE PEOPLE'S LIBERATION ARMY) 28 August 2013 (2013-08-28) entire document | 1-18 |
| A | CN 103145834 A (GUANGZHOU TAINUODI BIOTECHNOLOGY CO., LTD.) 12 June 2013 (2013-06-12) entire document | 1-18 |

☐ Further documents are listed in the continuation of Box C.    ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **12 December 2023** | **20 January 2024** |

| Name and mailing address of the ISA/CN | Authorized officer |
| --- | --- |
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/127070**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

International application No.

**PCT/CN2023/127070**

| Patent document cited in search report | | | Publication date (day/month/year) | Patent family member(s) | | | Publication date (day/month/year) |
|---|---|---|---|---|---|---|---|
| CN | 1839144 | A | 27 September 2006 | US | 2005033031 | A1 | 10 February 2005 |
| | | | | CA | 2533830 | A1 | 24 February 2005 |
| | | | | EP | 1651659 | A1 | 03 May 2006 |
| | | | | EP | 1651659 | A4 | 17 September 2008 |
| | | | | AU | 2003259718 | A1 | 07 March 2005 |
| | | | | WO | 2005016950 | A1 | 24 February 2005 |
| | | | | JP | 2007520991 | A | 02 August 2007 |
| CN | 103265631 | A | 28 August 2013 | CN | 103265631 | B | 08 July 2015 |
| CN | 103145834 | A | 12 June 2013 | CN | 103145834 | B | 18 March 2015 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202211335547